# FASCICULE DE BREVET EUROPEEN

(11) **EP 3 318 341 B1**
(45) Date de publication et mention de la délivrance du brevet: **26.02.2020**
(21) Numéro de dépôt: 17199392.6
(22) Date de dépôt: 31.10.2017
(51) Int. Cl.: B09B 3/00, A01C 3/00, A01C 3/02, B09B 5/00, C02F 3/28, C12M 1/107, C12M 1/16

(54) **DISPOSITIF ET PROCÉDÉ POUR LA METHANISATION PAR VOIE SECHE DE MATIERE ORGANIQUE.**
VORRICHTUNG UND VERFAHREN FÜR DIE METHANISIERUNG DURCH TROCKENVERFAHREN VON ORGANISCHEM MATERIAL.
DEVICE AND METHOD FOR DRY METHANISATION OF ORGANIC MATERIAL.

(30) Priorité: 03.11.2016 FR 1660645
(43) Date de publication de la demande: 09.05.2018
(73) Titulaire: EASYMETHA, 80440 Boves (FR)
(72) Inventeur: PEULTIER, Philippe, 80080 Amiens (FR)
(74) Mandataire: Cabinet Beau de Loménie

(56) Documents cités:
- FR-A1- 2 473 836
- FR-A1- 2 481 873
- US-A- 4 334 997
- US-A1- 2006 231 488

## Description

### Arrière-plan de l'invention

La présente invention concerne le domaine technique des dispositifs de méthanisation par voie sèche de matières organiques, ainsi que les procédés de méthanisation par voie sèche mettant en œuvre lesdits dispositifs.

La méthanisation est une transformation de la matière organique qui permet la production de biogaz et de digestat. La valorisation du biogaz permet d'obtenir de l'électricité, de la chaleur, du biométhane, utilisés par exemple comme carburants. La méthanisation permet également la production de digestats valorisables pouvant par exemple être utilisés comme fertilisants et ainsi être épandus.

La méthanisation est un processus naturel de recyclage de la matière organique fermentescible, dans un milieu sans oxygène (anaérobie), due à l'action de nombreux microorganismes qui transforment la matière organique en biogaz et en digestat. La méthanisation comprend quatre grandes étapes : l'hydrolyse, l'acidogénèse, l'acétogénèse et la méthanogénèse. La méthanisation conduit à la production d'un gaz, appelé biogaz, comprenant entre 40% et 70% (en volume) de méthane (CH₄) et, entre 30% et 50% (en volume) de dioxyde de carbone, qui est valorisable sous forme d'énergie en électricité grâce à un moteur de cogénération, en chaleur, en biocarburant ou qui peut être injecté dans le réseau de gaz naturel après une étape de purification devenant alors du bio-méthane.

La décomposition de la matière organique fournit également un produit riche en humus et partiellement stabilisé, appelé digestat, qu'il est ensuite possible de valoriser comme fertilisant naturel et amendement organique pour l'agriculture.

La méthanisation présente ainsi de nombreux avantages : la production locale et la vente d'électricité, de chaleur et de bio-méthane, la production d'un engrais renouvelable pour l'exploitation, la réalisation d'économies d'engrais grâce à une meilleure disponibilité de l'azote et du phosphore.

Différentes techniques de digestion existent : la digestion par voie liquide (également désignée sous le terme d'infiniment mélangé) et la digestion par voie sèche, notamment continue ou discontinue.

La digestion par voie liquide se fait généralement dans un digesteur comprenant une cuve de digestion en acier ou en béton, enterrée ou non, close hermétiquement et dont la température est maintenue soit à 37°C-39°C (régime mésophile), soit à 45-55°C (régime thermophile). La quantité de matière sèche dans la matière organique à traiter, laquelle matière organique comprend également des fluides, est généralement inférieure à 12% en masse. Le mélange à l'intérieur de la cuve est maintenu homogène grâce à un brassage continu qui a aussi pour fonction d'éviter la formation de la croûte en surface et la sédimentation des matières, et facilite également le dégazage.

La digestion par voie sèche continue ou discontinue se déroule en régime mésophile ou thermophile avec un temps de séjour au minimum de l'ordre de 20 jours.

Les matières organiques à digérer peuvent comprendre des végétaux, notamment des déchets végétaux (tontes, herbes, feuillages), bio-déchets alimentaires (biscuits, légumes,...), des lisiers ou des fumiers (paille et/ou menue paille éventuellement mélangée avec des lisiers et/ou déjections animales), par exemple des lisiers ou des fumiers de porcs, des lisiers ou des fumiers de bovins, des sous-produits agricoles, par exemple des résidus de céréales, de la menue paille, de la paille, des huiles végétales. Il a été constaté que la matière organique à digérer dite « sèche », c'est-à-dire comprenant au moins 15% en masse de matière sèche par rapport à la masse totale de ladite matière procure un meilleur rendement énergétique. En effet, la voie liquide décrite ci-dessus engendre des coûts de fonctionnement élevés en raison des machines de pompages et des agitateurs nécessaires à son bon fonctionnement. Ce type de digestion est adapté aux très grosses productions agricoles ou collectives mais convient moins à celles de petites ou moyennes tailles.

Le digestat est stockable et épandable avec un épandeur à fumier (une table d'épandage est préconisée). De plus, les coûts d'investissements sont globalement moins élevés que par la voie humide.

FR 2 481 873 et FR 2 495 887 ont pour objet des digesteurs dans lesquels la progression de la matière organique est assurée par une vis sans fin ou par des râteaux.

FR 2 475 524 a pour objet un digesteur dans lequel la progression de la matière organique est assurée par des pompes aptes également à l'homogénéiser. Le taux massique de matière sèche dans la matière organique n'est pas précisé mais doit être faible, et notamment inférieur ou égal à 10%, car elle peut être pompée de la zone de stockage pour alimenter la zone de digestion. Dans ce but, la matière organique est broyée puis mélangée avec de l'eau.

FR 2.473.836 a pour objet un dispositif de méthanisation de matière organique comprenant une zone de collecte et d'alimentation de la matière organique, un couloir de digestion, une zone de génération de gaz et une zone de collecte de gaz.

Les digesteurs par voie sèche ne doivent pas être confondus avec des digesteurs par voie liquide dans lesquels la matière organique peut être pompée, tel que par exemple le digesteur décrit dans US 4.334.997. En effet, dans ce digesteur, la matière organique à digérer est amenée dans la chambre de digestion ou évacuée de cette dernière par passage dans des conduits très étroits ce qui suppose que la matière organique à méthaniser soit très liquide, et donc pompable.

Les digesteurs agencés pour effectuer une méthanisation par voie sèche ont pour inconvénients que les dispositifs d'acheminement de la matière organique à digérer tout au long de la zone de digestion sont soumis à un milieu très agressif (acide et ce sous une chaleur de l'ordre de 37°C au moins) de sorte que ces derniers s'usent vite et nécessitent un entretien régulier, voire leurs renouvellements, ce qui peut s'avérer coûteux. De plus, ces digesteurs sont complexes et donc coûteux à fabriquer.

De nombreuses exploitations agricoles sont équipées d'aires paillées pour les animaux, il est donc nécessaire de curer régulièrement les élevages, ce qui peut être effectué quotidiennement à tous les quinze jours. A chaque curage, la matière organique collectée, comprenant du fumier est stockée dans une fumière.

Par ailleurs, le digesteur est alimenté régulièrement, de préférence quotidiennement, en matière organique à digérer. Il est donc nécessaire de collecter un volume donné de matière organique dans la fumière, correspondant à environ une journée de fonctionnement du digesteur pour alimenter le digesteur.

Le stockage de la matière organique à l'air libre dans la fumière a pour inconvénient qu'il prend de la place sur l'exploitation et que les conditions de stockage ne sont pas favorables à une bonne méthanisation ultérieure. En effet, la matière organique stockée à l'air libre dans la fumière commence à former du compost (dégradation aérobie) ce qui limite considérablement ses propriétés méthanogènes (dégradation anaérobie), des pertes d'environ 20% à 50% (en volume) de gaz produit par méthanisation sont observées (ces pertes étant calculées par rapport à la masse théorique de gaz qui devrait être produite).

Il existe ainsi un besoin pour des digesteurs de méthanisation par voie sèche simples à mettre en oeuvre, dont la maintenance est peu coûteuse et simplifiée, et capable de s'adapter à la taille des exploitations agricoles ainsi qu'à leur mode de fonctionnement (aires paillées et/ou logettes raclées).

Il existe également un besoin pour des digesteurs de méthanisation par voie sèche permettant un rendement amélioré, tout en simplifiant le fonctionnement de l'exploitation agricole.

### Objet et résumé de l'invention

La présente invention pallie tout ou partie des problèmes précités en ce qu'elle a pour objet, selon un premier aspect, un dispositif pour la méthanisation par voie sèche de matière organique comprenant avantageusement au moins un digesteur comprenant :
- au moins une zone de collecte et d'alimentation de la matière organique à digérer, de préférence comprenant du fumier comprenant de la paille et/ou de la menue paille ;
- un couloir de digestion comprenant :
   - une zone de génération de gaz destinée à recevoir la matière organique ayant été mouillée avec un fluide ; et
   - une zone de collecte du gaz disposée au-dessus de la zone de génération du gaz dans ledit couloir de digestion pour collecter le biogaz produit dans la zone de génération de gaz. Ledit couloir de digestion comprend une partie de toiture agencée pour former le plancher d'une zone préliminaire de réception de la matière organique, ladite zone de collecte et d'alimentation étant en contrebas de la partie de toiture en sorte que la matière organique lors de son avancée sur ladite partie de toiture tombe de la zone préliminaire de réception dans la zone de collecte et d'alimentation.

Avantageusement, il a été observé de manière surprenante que la matière organique brute comprenant du fumier, notamment disposée en forme de tas, arrosée avec un fluide (eau, déjections animales liquides, autres déchets organiques fluides, ou leur mélange), et ce tout en observant un taux de matière sèche élevé, adopte au fur et à mesure de sa maturation, et donc de sa digestion, un comportement rhéologique particulier lui permettant de se déplacer sous l'effet de son propre poids (déplacement gravitaire). On observe que la matière organique mouillée comprenant du fumier se comporte tel un solide en son centre et tel un liquide sur sa périphérie et donc en contact avec les parois du couloir de digestion ce qui permet son auto-déplacement.

Ce comportement de la matière organique n'est pas observable si cette dernière ne comprend pas de paille et/ou de menue paille et est par exemple constituée uniquement de lisiers et/ou de déjections animales et/ou de déchets verts.

Ledit au moins un digesteur selon l'invention utilise ainsi ce comportement de la matière organique mouillée avec un fluide comprenant du fumier afin d'obtenir le déplacement de cette dernière dans le digesteur entre son orifice d'admission et son orifice d'évacuation. Les dispositifs mécaniques d'avancement de la matière organique utilisés dans l'état de la technique, telles les vis sans fin ou les râteaux, nécessitant de l'énergie pour leur fonctionnement et coûteux pour leur maintenance, ne sont pas utiles dans le dispositif selon l'invention. Le déplacement de la matière organique mouillée avec un effluent dans le digesteur est obtenu sans l'aide de moyens mécaniques permettant son avancement.

Avantageusement, le digesteur selon l'invention peut traiter une matière organique brute qui n'a pas été au préalable broyée ou déchiquetée, en particulier qui n'a pas été mélangée avec de l'eau afin de la rendre pompable.

Avantageusement, il n'est plus nécessaire à l'éleveur de stocker la matière organique suite au curage dans une fumière puisque toute la matière organique collectée suite au curage est amenée directement dans la zone préliminaire de réception, laquelle est en liaison avec la zone de collecte et d'alimentation du couloir de digestion.

De plus, une partie, voire la totalité, de la toiture du couloir de digestion est utilisée pour former une zone préliminaire de réception de la matière organique, économisant ainsi de l'espace au sol sur l'exploitation, en particulier celui destiné à la fumière.

La zone de collecte et d'alimentation du couloir de digestion étant en contrebas de la zone préliminaire de réception, l'alimentation du couloir de digestion est facilitée puisque la matière organique tombe dans ladite zone de collecte.

De préférence, la zone préliminaire de réception comprend une ouverture d'évacuation de la matière organique dans ladite zone de collecte et d'alimentation, laquelle ouverture peut être fermée ou ouverte par un dispositif de fermeture et d'ouverture.

Le couloir de digestion comprend au moins une ouverture d'admission de la matière organique à digérer provenant de la zone de collecte et d'alimentation pour son admission dans la zone de génération de gaz, et au moins une ouverture d'évacuation de la matière organique digérée (ou digestat) pour son évacuation de la zone de génération de gaz (de préférence dans une zone d'évacuation et de stockage tampon définie ci-après), chacune desdites ouvertures pouvant l'une indépendamment de l'autre comprendre un dispositif de fermeture et d'ouverture.

Selon un mode de réalisation, la zone préliminaire de réception est une zone préliminaire de chargement.

Ce mode de réalisation est particulièrement adapté aux exploitations comprenant des logettes raclées par des moyens de curage en continu ou semi-continu. Dans l'état de la technique, pour un élevage comprenant des moyens de curage en continu ou semi-continu de la matière organique, la matière organique est convoyée vers une fumière par des moyens de collecte et de curage automatisés. Dans le cadre de la présente invention, les moyens de collecte et de curage automatisés sont agencés pour convoyer la matière organique, de manière continue ou semi-continue, directement dans la zone préliminaire de réception.

Dans ce cas, et de préférence, la matière organique est chauffée dans le couloir de digestion. Néanmoins, la zone préliminaire de réception peut comprendre des moyens de chauffage pour chauffer la matière organique et faire office également de zone préliminaire de chauffage. Il n'est pas en effet nécessaire de prévoir une zone préliminaire de chargement, distincte de la zone préliminaire de chauffage, permettant d'arroser la matière organique puisque cette dernière provenant des logettes comprend plus de liquides que la matière organique provenant des aires paillées.

Selon un autre mode de réalisation, la zone préliminaire de réception comprend une zone préliminaire de chargement et une zone préliminaire de chauffage, distincte de la zone préliminaire de chargement. La zone préliminaire de chauffage comprend des moyens de chauffage de la matière organique, et est disposée entre la zone préliminaire de chargement et la zone de collecte et d'alimentation du couloir de digestion.

Cette disposition est préférée lorsque la matière organique provient d'aires paillées, c'est-à-dire qu'elle est collectée de l'élevage ponctuellement, par exemple tous les jours (moyens de curage discontinu). Dans ce cas, la matière organique est disposée dans la zone préliminaire de chargement et est arrosée à l'aide d'un fluide, tel que défini dans le présent texte, afin de former un tas de volume homogène et s'adaptant au volume de ladite zone préliminaire de chargement.

Dans le cadre de la présente invention, la partie de toiture du couloir de digestion formant une partie du plancher de la zone préliminaire de réception est dans un matériau agencé pour supporter des charges de plusieurs dizaines de tonnes, et notamment en béton armé.

De préférence, le couloir de digestion est enterré en tout ou partie dans le sol en sorte que la zone préliminaire de réception soit au niveau du sol ou légèrement au-dessus de ce dernier, notamment à une hauteur inférieure ou égale à 2 m, en particulier inférieure ou égale à 1 m.

Selon un mode de réalisation, le digesteur selon l'invention comprend une zone d'évacuation et de stockage tampon de la matière organique digérée. Ledit couloir de digestion comprend dans ce cas au moins une ouverture d'évacuation de la matière organique digérée pour son évacuation de la zone de génération de gaz dans la zone d'évacuation et de stockage tampon, ladite ouverture d'évacuation comprenant un dispositif de fermeture et d'ouverture.

Avantageusement, la zone d'évacuation et de stockage tampon en sortie du couloir de digestion permet d'évacuer régulièrement dudit couloir de digestion la matière organique digérée ou digestat. Cette zone tampon permet non seulement de maîtriser/réguler la sortie de la matière organique digérée mais également de réguler la durée de méthanisation dans le couloir de digestion puisque ce dernier ne comprend aucun dispositif de déplacement de la matière organique.

On entend au sens de la présente invention par « fumier », de la paille et/ou de la menue paille, éventuellement mélangée(s) avec du lisier et/ou des déjections animales.

De préférence, la matière organique selon l'invention comprend du fumier éventuellement en mélange avec du lisier et/ou des déjections animales, en particulier issu(e)(s) des élevages bovins, porcins, ovins, volailles ou leur mélange ; et/ou des matières végétales, telles que des résidus de récolte ou autres déchets verts (tontes, feuillages, herbes,...), des déchets de céréales, de fruits et de légumes ; et/ou des déchets de l'industrie agro-alimentaire (biscuits,...) et/ou encore des collectivités (cantines,...). Encore de préférence, la matière organique selon l'invention est brute, notamment elle n'est pas broyée.

De préférence, la matière organique selon l'invention comprend au moins 30% de fumier en masse par rapport à sa masse totale, notamment au moins 50 % de fumier en masse par rapport à sa masse totale.

La matière organique mouillée avec un fluide selon l'invention ne peut pas être pompée car elle est trop riche en fibres, et présente un taux de matière sèche important.

Le taux de matière sèche est calculé en rapportant la masse totale de la matière organique mouillée par un fluide, par exemple 100g, (par exemple prélevée dans le couloir de digestion ou dans la zone préliminaire de réception avant ou après arrosage) sur la masse totale de matière sèche. La masse totale de la matière sèche peut être obtenue après avoir séché pendant plusieurs heures, notamment 6 h à 100°C en étuve avec extraction de vapeur, la matière organique mouillée.

De préférence, ledit fluide est de l'eau ou comprend des lisiers et/ou des déjections animales, notamment issus des élevages bovins, porcins, volailles ou ovins, ou encore leur mélange, et/ou des fluides issus de l'industrie agro-alimentaire, ces derniers pouvant être mélangés avec de l'eau.

De préférence, la matière organique est mouillée, notamment arrosée, par ledit fluide, lequel est de préférence à température ambiante. Ledit fluide peut être chauffé à une température supérieure ou égale à 30°C, encore de préférence supérieure ou égale à 35°C, en particulier inférieure ou égale à 60°C, plus particulièrement inférieure ou égale à 55°C.

Le mode de digestion selon l'invention peut être mésophile ou thermophile.

De préférence, la matière organique comprenant du fumier selon l'invention tient en tas lorsqu'elle est disposée dans la zone préliminaire de réception (ou directement dans la zone de collecte et d'alimentation) préalablement à son arrosage par un fluide. De préférence, la matière organique est disposée sous forme de dôme dans la zone préliminaire de réception puis arrosée jusqu'à ce que ledit tas, en particulier le dôme, s'abaisse.

La matière organique mouillée par ledit fluide est de préférence digérée pendant au moins sept jours dans le couloir de digestion, en particulier au moins pendant vingt jours.

De préférence, le taux de matière sèche de la matière organique (contenue dans le couloir de digestion ou dans la zone préliminaire de réception avant ou après arrosage), mouillée par un fluide, et apte à se déplacer de façon gravitaire est supérieur ou égal à 12 %, encore de préférence supérieur ou égal à 15 %, encore plus préférentiellement supérieur ou égal à 18 %.

Selon un mode de réalisation, le dispositif de fermeture et d'ouverture d'une ouverture d'admission et/ou d'une ouverture d'évacuation comprend un volet apte à pivoter autour d'un axe (F) sensiblement perpendiculaire au sens de déplacement (B) de la matière organique dans le couloir de digestion. De préférence, le sens de déplacement (B) de la matière organique dans le couloir de digestion est sensiblement parallèle à l'axe longitudinal (L) dudit couloir de digestion.

Selon un autre mode de réalisation, le dispositif de fermeture et d'ouverture d'une ouverture d'admission et/ou d'une ouverture d'évacuation comprend un organe de fermeture, par exemple une lame guillotine, agencé pour fermer une ouverture verticalement en se déplaçant dans un plan transversal, notamment perpendiculaire, à l'axe de déplacement (B) de la matière organique mouillée, en particulier perpendiculairement à l'axe longitudinal (L) du couloir de digestion.

Ledit dispositif de fermeture et d'ouverture peut être par exemple une vanne guillotine à fermeture et ouverture rapide.

Selon un mode de réalisation, la zone d'évacuation et de stockage tampon comprend également une ouverture d'évacuation de la matière organique digérée (digestat), laquelle est éventuellement pourvue d'un dispositif de fermeture et d'ouverture selon l'invention.

Selon un mode de réalisation, la zone d'évacuation et de stockage tampon est une fosse pourvue éventuellement d'un élément de toit amovible.

De préférence, le biogaz produit comprend environ de 40 % à 70 % en volume de méthane, de 30 % à 50 % en volume de dioxyde de carbone, et éventuellement des traces d'hydrogène sulfuré et d'eau.

Selon un mode de réalisation, le biogaz produit est épuré pour produire un biogaz comprenant au moins 97 % en volume de méthane.

Le biogaz produit comprenant du méthane à plus de 90 % en volume est ensuite compressé, puis odorisé afin de le rendre perceptible olfactivement. Ce biogaz ainsi traité peut ainsi fournir les réseaux d'alimentation classiques en gaz naturel (type GrDF).

Le digestat produit conserve les éléments fertilisants, et donc un potentiel humifère important.

Le couloir de digestion et éventuellement la zone d'évacuation et de stockage tampon, comprend/comprennent des moyens de collecte et d'acheminement du biogaz vers une zone de stockage du biogaz.

Dans une sous-variante de réalisation, le couloir de digestion comprend au moins deux parois latérales (gauche et droite) et une paroi de fond, et un moyen de raclement d'au moins l'une desdites parois latérales et de fond disposé librement.

De préférence, le moyen de raclement comprend au moins une grille disposée librement dans la matière organique et se déplaçant le long du couloir de digestion sous l'effet du déplacement libre de la matière organique.

Ledit moyen de raclement peut être disposé, notamment à plat, dans la zone de collecte et d'alimentation puis entraîné par la matière organique à travers au moins une ouverture d'admission dudit couloir de digestion puis dans ce dernier.

Ce moyen de raclement, en particulier lorsqu'il s'agit d'une grille a pour effet d'homogénéiser l'avancement de la matière organique dans le couloir de digestion et d'éviter que la matière organique ne colle aux parois latérales et de fond du couloir. Ladite au moins une grille permet également de dégazer la matière organique.

Selon un mode de réalisation, les parois latérales gauche et droite du couloir de digestion sont parallèles selon tout ou partie de leur longueur et/ou convergent selon une partie de leur longueur vers l'ouverture d'évacuation. Cette dernière disposition favorise le cisaillement de la matière organique mouillée et donc son homogénéisation.

De préférence, le moyen de raclement est dimensionné par rapport aux dimensions du couloir de digestion en sorte de racler au moins l'une, voire l'ensemble, des parois latérales et de fond du couloir de digestion.

De préférence, le moyen de raclement, en particulier chaque grille selon l'invention, comprend une partie supérieure et une partie inférieure montées sur un ressort de rappel en sorte d'être maintenues l'une contre l'autre au repos et d'être aptes à s'écarter l'une de l'autre sous l'effet d'une contrainte jusqu'à tenir dans un même plan en pivotant autour de l'axe transversal séparant la partie supérieure de la partie inférieure.

Dans un second mode de réalisation, inverse au mode précédent, le moyen de raclement comprend au moins une grille, laquelle grille comprend une partie supérieure et une partie inférieure montées l'une par rapport à l'autre à l'aide d'un dispositif de rappel, tel un ressort de rappel, en sorte qu'en position déployée, et donc au repos, les parties supérieure et inférieure soient sensiblement dans un même plan, et qu'en position pliée, la partie supérieure soit apte à pivoter et se rabattre vers la partie inférieure.

Dans un mode de réalisation, le dispositif de méthanisation comprend une ouverture de récupération dudit au moins un moyen de raclement, disposée près de ladite au moins une ouverture d'évacuation du couloir de digestion.

Ledit moyen de raclement est ainsi introduit dans le couloir de digestion en disposant ce dernier dans la zone de collecte et d'alimentation.

De préférence, les ouvertures de la grille ont au moins une dimension supérieure à un centimètre.

De préférence, le dispositif selon l'invention comprend au moins deux moyens de raclement, encore de préférence au moins quatre moyens de raclement.

Dans une sous-variante de réalisation, le dispositif de méthanisation comprend des moyens de chauffage disposés dans au moins l'une desdites parois latérales et de fond, et un moteur de cogénération configuré pour être alimenté au moins en partie par le biogaz produit dans la zone de collecte et pour alimenter en chaleur lesdits moyens de chauffage.

De préférence, le module de cogénération comprend un moteur entraînant un alternateur-générateur de courant électrique. De préférence, le moteur de cogénération est un moteur à gaz.

Dans une sous-variante de réalisation, le dispositif de méthanisation comprend une ouverture d'admission dudit moyen de raclement.

L'ouverture d'admission et/ou l'ouverture d'évacuation du moyen de raclement est/sont de préférence agencée(s) dans la partie de toiture du couloir de digestion.

Dans une sous-variante de réalisation, le couloir de digestion comprend au moins deux, de préférence au moins trois ouvertures d'évacuation alignées verticalement.

Dans une variante, la zone de collecte et d'alimentation, le couloir de digestion, et la zone d'évacuation et de stockage tampon, sont au moins partiellement enterrés.

De préférence, la zone de collecte et d'alimentation, le couloir de digestion (y compris la partie de toiture), et la zone d'évacuation et de stockage tampon sont au moins en partie en béton, ledit béton est de préférence isolé thermiquement L'enterrement au moins partiel desdites zones du dispositif de méthanisation et du couloir de digestion limitent également les pertes de chaleur.

Dans une variante, le dispositif de méthanisation comprend selon une portion déterminée de la longueur du couloir de digestion, une zone d'évent laquelle est recouverte par au moins une membrane imperméable au biogaz, de préférence ladite membrane est recouverte d'une membrane de protection.

Ladite zone d'évent permet au gaz de s'échapper en cas d'explosion en créant une zone de « faiblesse » orientée dans le dispositif de méthanisation. La ou les membrane(s) superposée(s) se déchirera/déchireront sous l'effet de l'explosion sans endommager le restant de la structure du dispositif de méthanisation.

Ladite zone d'évent s'étend sur au moins un tiers de la longueur du couloir de digestion.

Dans une sous-variante de réalisation, le dispositif de méthanisation comprend au moins deux digesteurs, lesdits deux digesteurs ayant au moins une paroi latérale commune.

Cette disposition favorise les économies d'énergie pour le chauffage de la matière organique. Par ailleurs, l'entretien du dispositif selon l'invention est facilité puisqu'il est possible d'accéder à un couloir de digestion sans stopper le travail d'un autre couloir de digestion parallèle.

Enfin, cette disposition permet de s'adapter à la taille des exploitations agricoles ou équivalentes, en particulier si leurs productions de matières organiques ne sont pas constantes.

Les couloirs de digestion sont de préférence disposés en parallèle.

Dans une variante de réalisation, la zone préliminaire de réception comprend une zone préliminaire de chauffage comprenant des moyens de chauffage pour le chauffage de la matière organique.

La zone préliminaire de chauffage permet de préparer la matière organique avant sa méthanisation dans le couloir de digestion, de préférence la matière organique est chauffée à une température supérieure ou égale à 38°C pour que la méthanisation puisse commencer efficacement.

La zone préliminaire de réception, et donc les zones préliminaires de chargement et de chauffage, comprennent des parois latérales (gauche et droite) et une paroi de fond formée par ladite partie de toiture.

Les moyens de chauffage préliminaires peuvent être agencés dans au moins l'une des parois latérales ou la partie de toiture, de préférence dans la paroi de toiture formant le plancher, de la zone préliminaire de chauffage.

Dans une sous-variante de réalisation, la zone préliminaire de réception comprend une zone préliminaire de chargement, et la zone préliminaire de chauffage comprend au moins une ouverture d'admission de la matière organique provenant de la zone préliminaire de chargement, et au moins une ouverture d'évacuation de la matière organique vers la zone de collecte et d'alimentation, chacune des ouvertures d'admission et d'évacuation comprend un dispositif d'ouverture et de fermeture.

Lorsque la matière organique est issue d'aires paillées, le fumier est collecté lorsque l'éleveur cure lesdites aires paillées, ce qui peut être effectué tous les jours ou selon un laps de temps plus long selon les besoins. La zone préliminaire de chargement fait alors office de zone stockage dans laquelle le fumier est de préférence arrosé en sorte de limiter la fermentation aérobie.

La zone de chargement permet de préparer la matière organique en ajoutant du fluide, c'est-à-dire en quantité suffisante pour boucher les interstices dans la matière mais non pour mettre en suspension cette dernière (telle que cela est le cas en méthanisation par voie liquide).

Dans le cas des exploitations comprenant des logettes raclées par des moyens de collecte et de convoyage en continu ou semi-continu, la zone préliminaire de chargement ne fait pas office de zone stockage puisque la matière organique est convoyée plusieurs fois par jour dans la zone préliminaire de chargement. Dans ce cas, il n'est pas nécessaire d'arroser le fumier pour éviter une fermentation aérobie, la matière organique étant rapidement transférée dans la zone de collecte et d'alimentation puis dans le couloir de digestion. Néanmoins, l'arrosage de la matière organique peut être envisagé si l'on souhaite éviter une fermentation aérobie et retarder le transfert de la matière organique dans la zone préliminaire de chauffage.

Dans une variante de réalisation, la zone préliminaire de réception comprend un dispositif poussoir agencé pour pousser la matière organique disposée dans la zone de collecte et d'alimentation ou pour pousser la matière organique, stockée dans la zone préliminaire de chargement, dans la zone préliminaire de chauffage, la matière organique étant poussée selon un sens de déplacement A opposé au sens de déplacement B de la matière organique dans le couloir de digestion.

Le dispositif poussoir est préféré lorsque la matière organique est issue d'aires paillées.

Le dispositif poussoir est de préférence actionné par des moyens de commande permettant de sélectionner son avancée dans la zone préliminaire de chargement pour sélectionner un volume déterminé de matière organique à pousser vers la zone préliminaire de chauffage. Le dispositif poussoir avance ainsi en sorte de sélectionner un volume v de matière organique correspondant au volume totale V = n ^{∗} v. Le nombre entier n peut correspondre au nombre de jours d'un cycle de curage, par exemple n vaut 7.

Dans une variante de réalisation, le dispositif poussoir comprend une plaque montée sur un organe poussoir (par exemple un vérin ou un treuil). Ledit dispositif poussoir est agencé en sorte de déplacer en translation (de préférence axialement) ladite plaque par rapport à la partie de toiture. Le plan P1 de ladite plaque étant sensiblement perpendiculaire au plan P3 principal de ladite partie de toiture dans la zone préliminaire de chargement.

Dans une variante de réalisation, le dispositif poussoir est actionné par des moyens de commande permettant de pousser tout ou partie du volume de la matière organique contenue dans la zone préliminaire de réception ou contenue dans la zone préliminaire de chargement.

Un volume déterminé de matière organique est poussé de la zone préliminaire de réception vers la zone de collecte et d'alimentation, ou selon un mode préféré, de la zone préliminaire de chargement vers la zone préliminaire de chauffage.

Dans une variante de réalisation, la partie de toiture de la zone préliminaire de réception, en particulier la partie de toiture dans la zone préliminaire de chauffage, comprend au moins une portion inclinée vers la zone de collecte et d'alimentation.

De préférence, ladite au moins une portion inclinée de la partie de toiture dans la zone préliminaire de réception, en particulier dans la zone préliminaire de chauffage, est dans un plan P2 sécant au plan P3 principale de ladite partie de toiture (notamment au plan P3 de la partie de toiture dans la zone préliminaire de chargement).

Dans une variante de réalisation, les moyens de chauffage de la zone préliminaire de chauffage comprennent des moyens d'alimentation en eau chaude, en particulier issue de la cogénération.

La présente invention a pour objet selon un deuxième aspect, un procédé de méthanisation par voie sèche de matière organique comprenant avantageusement les étapes suivantes, notamment effectuées à l'aide du dispositif de méthanisation décrit selon l'un(e) quelconque des variantes ou modes de réalisation décrits en référence au premier aspect :
a) une étape de réception de la matière organique à digérer (en particulier comprenant du fumier comprenant de la paille et/ou de la menue paille) dans une zone préliminaire de réception ;
b) une étape de transfert de la matière organique de la zone préliminaire de réception dans une zone de collecte et d'alimentation ; et
c) une étape de transfert de la matière organique de la zone de collecte et d'alimentation vers un couloir de digestion ayant au moins une ouverture d'admission de la matière organique et au moins une ouverture d'évacuation de la matière organique, l'avancée de la matière organique dans le couloir de digestion entre les ouvertures d'admission et d'évacuation est obtenue par le déplacement gravitaire de la matière organique, le couloir de digestion comprenant un dispositif de fermeture et d'ouverture de ladite au moins une ouverture d'évacuation ; et
d) une étape de collecte du biogaz produit.

Avantageusement, le couloir de digestion comprend une partie de toiture agencée pour former le plancher de la zone préliminaire de réception de la matière organique, ladite zone de collecte et d'alimentation étant en contrebas de la partie de toiture en sorte que la matière organique puisse tomber, lors de son avancée, de la zone préliminaire de réception dans la zone de collecte et d'alimentation.

De préférence, la zone préliminaire de réception comprend une zone préliminaire de chargement et une zone préliminaire de chauffage, toutes deux distinctes.

La matière organique est ainsi, de préférence, chargée dans la zone préliminaire de chargement puis transférée, notamment poussée par un dispositif poussoir, de préférence selon un volume déterminé, dans la zone préliminaire de chauffage pour son chauffage, et enfin transférée dans la zone de collecte et d'alimentation en tombant dans cette dernière.

Dans une variante de réalisation, ledit procédé comprend une étape d'arrosage de la matière organique disposée dans la zone préliminaire de réception à l'aide d'un fluide, en particulier dans la zone préliminaire de chargement.

Ledit fluide est de préférence à température ambiante (supérieure à 0°C et inférieure ou égale à 40°C, notamment supérieure ou égale à 10°C et inférieure ou égale à 30°C. Ledit fluide peut être chauffé également, notamment à une température supérieure ou égale à 38°C, et inférieure ou égale à 60°C.

Avantageusement, l'étape d'arrosage à l'aide d'un fluide de la matière organique à méthaniser dans la zone préliminaire de réception permet : de remplir la porosité dans la matière organique à méthaniser puisque la matière sèche, c'est-à-dire notamment la paille et/ou la menue paille, est présente en quantité importante, afin de conserver les propriétés méthanogènes de la matière à méthaniser ; et d'obtenir l'effondrement du tas facilitant ainsi la poussée de la matière organique par le dispositif poussoir, et donc le transfert et le remplissage de la zone préliminaire de chauffage et/ou de la zone de collecte et d'alimentation en entrée du couloir de digestion.

Dans une variante de réalisation, ledit procédé comprend une étape de chauffage préliminaire de la matière organique dans la zone préliminaire de réception, de préférence l'étape préliminaire de chauffage est effectuée après l'étape d'arrosage, notamment dans la zone préliminaire de chauffage.

La matière organique est de préférence chauffée à une température supérieure ou égale à 38°C, et inférieure ou égale à 55°C, en particulier inférieure ou égale à 50°C, plus particulièrement inférieure ou égale à 45°C.

Le chauffage préliminaire de la matière à méthaniser dans un volume restreint, en particulier dans la zone préliminaire de chauffage, favorise l'homogénéité de la température dans la masse, permet également à la matière entrante dans le digesteur d'être à la température idéale pour la fermentation méthanogène.

Dans une variante de réalisation, le procédé de méthanisation comprend une étape d'arrosage de la matière organique disposée dans la zone préliminaire de chargement à l'aide d'un fluide.

Dans une variante de réalisation, le procédé de méthanisation comprend une étape de chauffage préliminaire de la matière organique dans une zone préliminaire de chauffage dont le plancher est formé également par la partie de toiture du couloir de digestion.

Dans une variante, le procédé comprend une étape de transfert de la matière organique digérée (digestat) du couloir de digestion vers une zone d'évacuation et de stockage tampon.

Les modes réalisation, définitions et (sous)variantes de réalisation du dispositif de méthanisation selon l'invention peuvent être appliqués indépendamment les uns des autres au procédé de méthanisation selon l'invention.

### Brève description des dessins

La présente invention sera mieux comprise à la lecture des exemples de réalisation décrits ci-après et cités à titre non limitatifs, illustrés par les figures suivantes annexées à la présente, et dans lesquelles :
- La figure **1** est une représentation schématique et vue de dessus d'un premier exemple de dispositif de méthanisation selon l'invention ;
- La figure **2** est une représentation schématique selon le plan de coupe II-II représenté à la figure **1** du premier exemple de dispositif de méthanisation ;
- Les figures **3** à **6** représentent de manière schématique les différentes étapes d'un procédé de méthanisation selon l'invention mettant en œuvre le dispositif de méthanisation représenté sur les figures **1** et **2** ; et
- La figure **7** représente de manière schématique un second exemple de digesteur selon l'invention.

### Description détaillée des exemples de réalisation

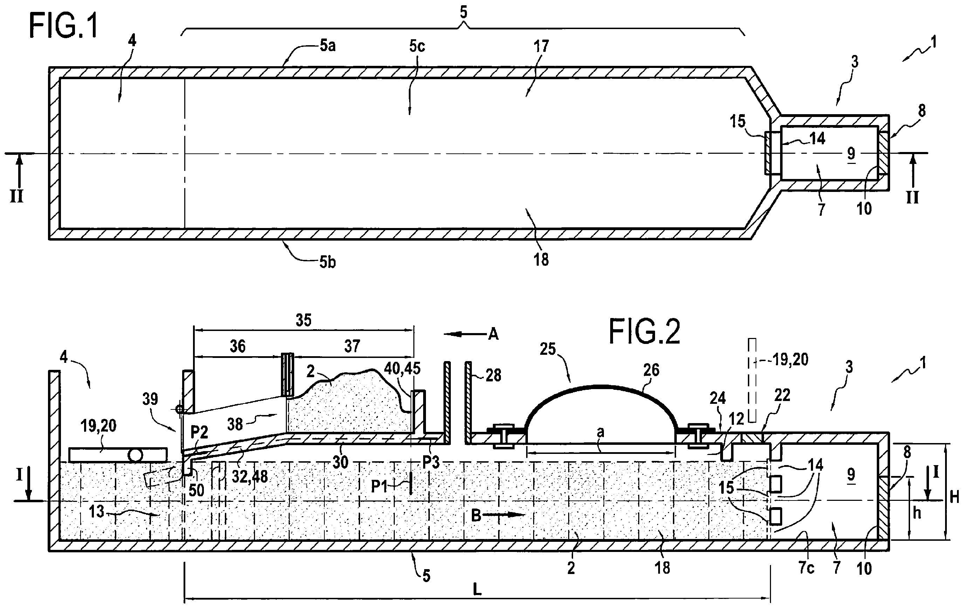

Le premier exemple de dispositif de méthanisation **1** par voie sèche de matière organique **2** selon l'invention, représenté aux figures **1** et **2**, comprend un digesteur **3** comprenant une zone de collecte et d'alimentation **4** de la matière organique **2** à digérer, notamment apte à tenir en tas, un couloir de digestion **5** et une zone d'évacuation et de stockage tampon **7** de la matière organique digérée formant un digestat **9.** Ledit couloir de digestion **5** comprend une zone de génération de gaz **11** destinée à recevoir la matière organique **2** ayant été mouillée avec un fluide et une zone de collecte du gaz **12** disposée au-dessus de la zone de génération du gaz **11** dans ledit couloir de digestion **5** pour collecter le biogaz produit dans la zone de génération de gaz **11.** Ledit couloir de digestion **5** comprend également au moins une ouverture d'admission **13** de la matière organique **2** à digérer pour son admission de la zone d'alimentation et de collecte **4** dans la zone de génération de gaz **11**, ladite ouverture d'admission **13** comprenant éventuellement un dispositif de fermeture et d'ouverture (non représenté). Ledit couloir de digestion **5** comprend en outre trois ouvertures d'évacuation **14**, alignées verticalement, de la matière organique digérée **9** pour son évacuation de la zone de génération de gaz **11** dans la zone d'évacuation et de stockage tampon **7**, lesdits orifices d'évacuation **14** comprenant un dispositif de fermeture et d'ouverture **15** permettant la fermeture ou l'ouverture simultanée desdites trois ouvertures **14** ou de manière différenciée. Cette disposition permet avantageusement une sortie de manière homogène de la matière organique digérée.

Selon un mode de réalisation, la zone d'évacuation et de stockage tampon **7** comprend également une ouverture d'évacuation **8** de la matière organique digérée **9** (digestat) pourvue d'un dispositif de fermeture et d'ouverture **10**, notamment identique au dispositif de fermeture et d'ouverture **15** de ladite au moins une ouverture d'évacuation **14** du couloir de digestion **5.**

L'ouverture d'évacuation **8** de la matière organique digérée **9** s'étend du fond **7c** de ladite zone d'évacuation et de stockage tampon **7.** Cette disposition facilite la sortie et donc l'évacuation de la matière organique digérée **9** en évitant que cette dernière ne sédimente en certains endroits de la zone d'évacuation et de stockage tampon **7.**

Le couloir de digestion **5** comprend des moyens de chauffage pour le chauffage de la matière organique **2.**

Le couloir de digestion **5** comprend une partie de toiture **30** agencée pour former le plancher **32** d'une zone préliminaire de réception **37** de la matière organique **2**, ladite zone de collecte et d'alimentation **4** étant en contrebas de la partie de toiture **30** en sorte que la matière organique **2** lors de son avancée dans la zone préliminaire de réception **35** tombe de ladite zone préliminaire de réception **35** dans la zone de collecte et d'alimentation **4.**

La zone préliminaire de réception **35** comprend une zone préliminaire de chauffage **36** comprenant des moyens de chauffage pour le chauffage de la matière organique **2** et une zone préliminaire de chargement **37.** La zone préliminaire de chauffage **36** comprend au moins une ouverture d'admission **38** de la matière organique **2** provenant de la zone préliminaire de chargement **37**, et au moins une ouverture d'évacuation **39** de la matière organique **2** vers la zone de collecte et d'alimentation **4**, chacune des ouvertures d'admission **38** et d'évacuation **39** comprend un dispositif d'ouverture et de fermeture.

La zone préliminaire de réception **35** comprend un dispositif poussoir **40** agencé pour pousser la matière organique **2** stockée dans la zone préliminaire de chargement **37**, dans la zone préliminaire de chauffage **36**, la matière organique **2** étant poussée selon un sens de déplacement A opposé au sens de déplacement B de la matière organique dans le couloir de digestion.

Le dispositif poussoir **40** comprend une plaque **45** montée sur un organe poussoir (non représenté), ledit dispositif poussoir **40** est agencé en sorte de déplacer ladite plaque **45** en translation axialement par rapport à la partie de toiture **30.** Le dispositif poussoir **40** est actionné par des moyens de commande permettant de pousser tout ou partie du volume de la matière organique **2** contenue dans la zone préliminaire de chargement **37.**

La partie de toiture **30** dans la zone préliminaire de chauffage **36** comprend une portion inclinée **48** vers la zone de collecte et d'alimentation **4**.

Le couloir de digestion **5** est défini par des parois latérales gauche **5a** et droite **5b** et une paroi de fond **5c**, et comprend au moins un moyen de raclement **19** d'au moins l'une desdites parois latérales **5a**,**5b** ou de fond **5c** disposé librement.

Le digesteur **3** comprend des moyens de chauffage disposés dans au moins l'une desdites parois latérales **5a,5b** et de fond **5c**, ici dans chacune des parois latérales **5a,5b** et de fond **5c**. Le digesteur **3** comprend un module de cogénération (non représenté) comprenant un moteur de cogénération configuré pour être alimenté au moins en partie par le biogaz produit dans la zone de collecte **12** et pour alimenter en chaleur lesdits moyens de chauffage.

Le digesteur **3** comprend une ouverture de récupération **22** du moyen de raclement **19**, disposée près de l'ouverture d'évacuation **14** du couloir de digestion **5**.

La partie supérieure du couloir de digestion **5** est fermée de manière étanche à l'aide d'une partie de toiture **30**. Ladite ouverture de récupération **22** du moyen de raclement **19** est disposée dans ladite partie de toiture **30**.

De préférence, la zone de collecte et d'alimentation **4**, le couloir de digestion **5**, et la zone de stockage tampon **7** sont en béton, notamment isolé thermiquement, et sont enterrés.

Le dispositif **3** comprend une cloison de déviation **50** disposée entre la zone de collecte et d'alimentation **4** et le couloir de digestion **5**, et délimitant ainsi l'ouverture d'admission **13**.

Tel que cela est visible à la figure **2**, le digesteur **3** comprend selon une portion déterminée de la longueur L du couloir de digestion **5**, une zone d'évent **25** laquelle est recouverte par au moins une membrane imperméable au biogaz **26.**

A titre d'exemple, et de manière purement indicative et non limitative, la longueur L peut être d'environ 25 mètres, la hauteur H peut être d'environ 4-5 mètres et la hauteur h peut être d'environ 2 mètres. La taille d'une ouverture d'évacuation **14** peut être de l'ordre de 60 cm de diamètre.

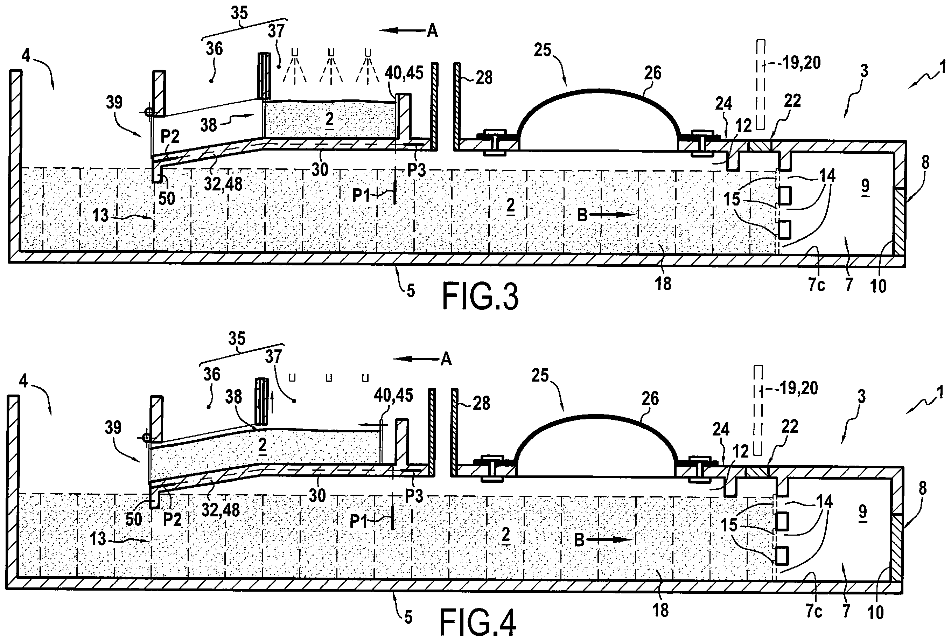

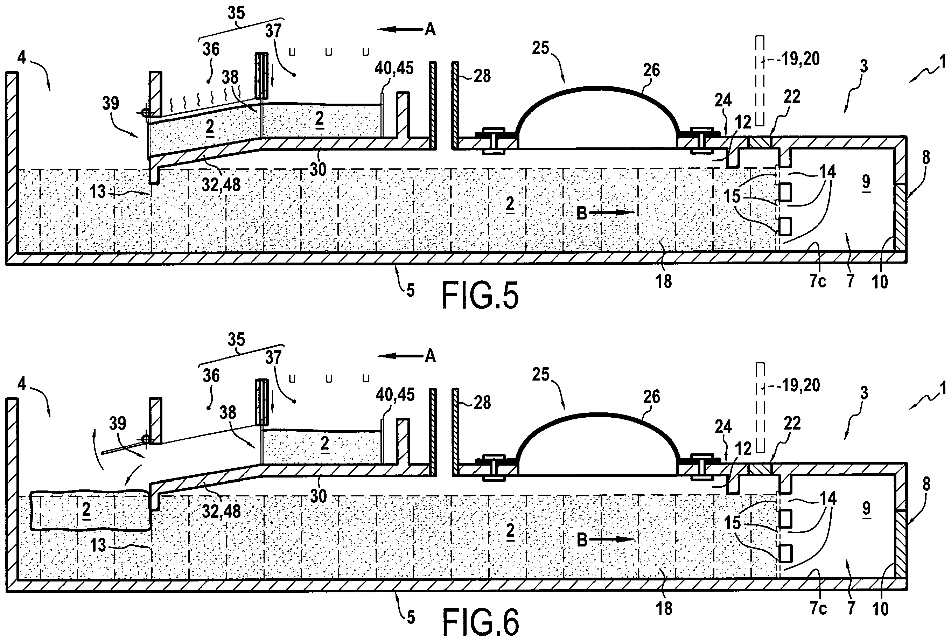

En fonctionnement, la matière organique **2** comprenant du fumier issu d'une opération de curage est chargée directement dans la zone préliminaire de chargement **37** en sorte de former un tas, notamment ayant une forme de dôme. Puis, la matière organique **2** est arrosée à l'aide d'un fluide (voir figure **3**), à la température ambiante, en particulier jusqu'à écroulement du tas dans ladite zone de chargement **37.** Le dispositif de fermeture et d'ouverture de l'ouverture d'admission **38** est ouvert, puis un volume donné de la matière organique **2** est poussé par l'intermédiaire du dispositif poussoir **40** dans la zone préliminaire de chauffage **36** tel que représenté à la figure **4****.** Le dispositif poussoir **40** déplace la plaque **45** par l'intermédiaire d'un organe poussoir selon une direction A, opposée au sens de circulation B de la matière organique dans le couloir de digestion **5.** La plaque **45** est déplacée axialement en sorte de pousser une quantité de matière organique **2** déterminée dans la zone préliminaire de chauffage **36** correspondant par exemple à une durée de fonctionnement précise du digesteur, par exemple pour une journée. Le dispositif de fermeture et d'ouverture de l'ouverture d'évacuation **38** est fermé. L'ouverture d'évacuation **39** reste fermée également tel que représenté à la figure **5****.** La matière organique **2** est alors chauffée à une température de l'ordre de 40°C tel que représentée à la figure **5****,** notamment durant la période de stockage de la matière organique **2** dans la zone préliminaire de chauffage **36** avant son transfert dans la zone **4.** Puis, le dispositif de fermeture et d'ouverture de l'ouverture d'évacuation **39** de la zone préliminaire de chauffage **36** est ouvert en sorte que la matière organique **2** tombe dans la zone de collecte et d'alimentation (voir figure **6**) puis avance sous l'effet de son propre poids à travers l'ouverture d'admission **13** dans le couloir de digestion **5**, en particulier dans la zone de génération **11.** Les parois latérales gauche **5a** et droite **5b**, la paroi de fond **5c**, et la cloison de séparation **16** peuvent être éventuellement chauffées en sorte que la matière organique **2** soit maintenue en température, de préférence à une température supérieure ou égale à 35°C, en particulier au moins de l'ordre de 37°C. Le chauffage du couloir de digestion **5** est alimenté en tout ou partie par le module de cogénération (non représenté) alimenté en tout ou partie par le biogaz produit par le dispositif de méthanisation **1.** La matière organique **2** mouillée est transférée sous l'effet de son propre poids entre les ouvertures d'admission **13** et d'évacuation **14** dans le couloir de digestion **5** selon l'axe (B), lequel est sensiblement parallèle à l'axe longitudinal (L) du couloir de digestion **5.** Le dispositif de fermeture et d'ouverture **15** ferme les ouvertures d'évacuation **14.** La matière organique **2**, mouillée avec ledit fluide, est digérée pendant au moins 20 jours dans le couloir de digestion **5**, le gaz produit est collecté dans la zone de collecte du gaz **11** par l'intermédiaire du conduit **28** représenté à la figure **2**, lequel est indirectement relié au module de cogénération. Lorsque la matière organique **2** se déplace, elle entraîne les grilles **20** permettant le nettoyage des parois **5a,5b,5c** du couloir de digestion **5** mais aussi l'homogénéisation de la matière organique **2** et son dégazage. L'opérateur peut facilement enlever les grilles **20** via l'ouverture de récupération **22** et/ou en ajouter par la zone de stockage et d'alimentation **4.**

Lorsque la matière organique est digérée, le digestat **9** est évacué par les ouvertures d'évacuation **14** vers la zone de stockage tampon **7**, après ouverture du dispositif de fermeture et d'ouverture **15**, lequel dispositif **15** comprend un organe de fermeture, par exemple une lame guillotine, apte à fermer les trois orifices d'évacuation, en se déplaçant selon une direction transversale, notamment perpendiculaire, à l'axe de déplacement (B) de la matière organique **2** mouillée, en particulier perpendiculairement à l'axe longitudinal (L) du couloir de digestion **5.**

Une fois la zone de stockage tampon **7** remplie du digestat **9**, le dispositif de fermeture et d'ouverture **10** est ouvert ce qui permet de récupérer le digestat **9.** La zone de stockage tampon **7** permet de contrôler le déplacement de la matière organique **2** et donc l'avancée de la matière organique **2** dans le couloir de digestion **5** et son degré de digestion en l'absence de moyens mécaniques d'avancée de la matière organique **2.** La zone d'évacuation et de stockage tampon **7** permet également de contrôler la poussée de la matière organique **2** mouillée sur les parois du couloir de digestion et de celles de la cloison de séparation **16** en ouvrant les orifices d'évacuation **14.**

Les opérations illustrées sur les figures **3** à **6** sont répétées. Le dispositif poussoir **40** de préférence pousse au fur et à mesure un volume déterminé journalier de la matière organique dans la zone préliminaire de chargement **37.**

Dans le cas par exemple d'exploitations en aires paillées, l'élevage est curé tous les **7** jours, la matière organique **2** est directement chargée dans la zone préliminaire de chargement **37.** Le dispositif poussoir **40** est alors actionné par des moyens de commande une fois par jour permettant l'avancement de la matière organique **2** en sorte de pousser une quantité de matière organique correspondant à 1/7 du volume de la zone préliminaire de chargement. La plaque **45** avance donc tous les jours progressivement vers l'ouverture d'évacuation. Une fois que toute la matière organique **2** a été transférée dans la zone préliminaire de chauffage **36**, le dispositif poussoir **40** est reculé dans sa position initiale, afin qu'un nouveau volume de matière organique soit chargé dans la zone préliminaire de chargement.

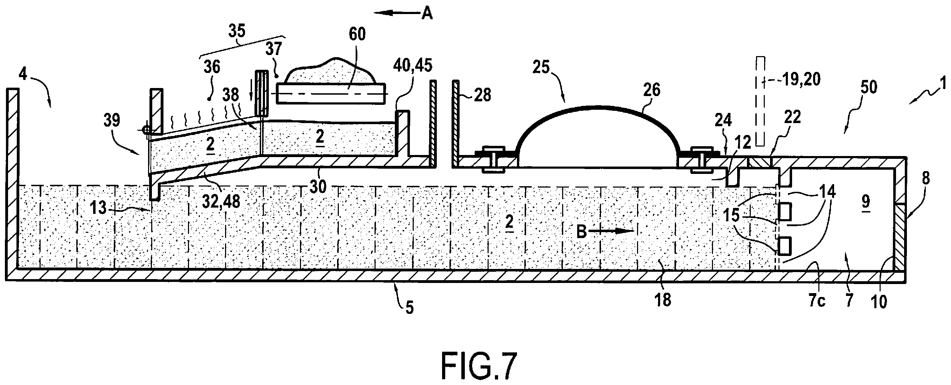

La figure **7** représente une variante du digesteur **1** représentée sur les figures précédentes. Ce digesteur **50** ne sera décrit que de par ses différences avec le digesteur **1.** Les références des éléments du digesteur **1** sont reprises sur la figure **7** car ces derniers sont identiques à ceux décrits dans les figures **1** à **6****.**

Le digesteur **50** est particulièrement adapté aux exploitations comprenant des logettes dont le curage est assuré de manière continue ou semi-continue par l'intermédiaire de moyens de collecte et de convoyage de la matière organique, en particulier du fumier, à partir de l'élevage. Les dits moyens peuvent être par exemple un évacuateur à chaînes de fumier **60**, tel que celui représenté schématiquement à la figure 7.

Dans ce cas, le procédé selon l'invention ne comprend pas de préférence d'étape d'arrosage. En effet, le fumier alimente le digesteur 50 de manière continue ou semi-continue, il n'est donc pas nécessaire d'arroser le fumier pour éviter une fermentation aérobie pendant son stockage. La zone préliminaire de chargement ne fait pas office dans ce cas de zone préliminaire de stockage.

Une opération préliminaire de chauffage est de préférence effectuée. Enfin, la zone préliminaire de réception **35** ne comprend pas dans cet exemple précis de dispositif poussoir.

## Revendications

1. Dispositif (1) pour la méthanisation par voie sèche de matière organique (2), comprenant au moins un digesteur (3) comprenant:
- au moins une zone de collecte et d'alimentation (4) de la matière organique (2) à digérer;
- un couloir de digestion (5) comprenant :
- une zone de génération de gaz (11) destinée à recevoir la matière organique (2) ayant été mouillée avec un fluide ; et
- une zone de collecte du gaz (12) disposée au-dessus de la zone de génération du gaz (11) dans ledit couloir de digestion (5) pour collecter le biogaz produit dans la zone de génération de gaz (11) ;
**caractérisé en ce que** le couloir de digestion comprend une partie de toiture (30) agencée pour former le plancher (32) d'une zone préliminaire de réception (35) de la matière organique, ladite zone de collecte et d'alimentation (4) étant en contrebas de la partie de toiture (30) en sorte que la matière organique lors de son avancée dans ladite zone préliminaire de réception (35) tombe de ladite zone préliminaire de réception (35) dans la zone de collecte et d'alimentation (4).

2. Dispositif (1) selon la revendication 1, **caractérisé en ce que** la zone préliminaire de réception (35) comprend une zone préliminaire de chauffage (36) comprenant des moyens de chauffage pour le chauffage de la matière organique (2).

3. Dispositif (1) selon la revendication 2, **caractérisé en ce que** la zone préliminaire de réception (35) comprend une zone préliminaire de chargement (37), et **en ce que** la zone préliminaire de chauffage (36) comprend au moins une ouverture d'admission (38) de la matière organique (2) provenant de la zone préliminaire de chargement (37), et au moins une ouverture d'évacuation (39) de la matière organique (2) vers la zone de collecte et d'alimentation (4), chacune des ouvertures d'admission (38) et d'évacuation (39) comprend des moyens d'ouverture et de fermeture.

4. Dispositif (1) selon l'une quelconque des revendications 1 à 3, **caractérisé en ce que** la zone préliminaire de réception (35) comprend un dispositif poussoir agencé pour pousser (40) la matière organique (2) dans la zone de collecte et d'alimentation (4) ou pour pousser la matière organique (2), stockée dans la zone préliminaire de chargement (37), dans la zone préliminaire de chauffage (36), la matière organique (2) étant poussée selon un sens de déplacement A opposé au sens de déplacement B de la matière organique (2) dans le couloir de digestion.

5. Dispositif (1) selon la revendication 4, **caractérisé en ce que** le dispositif poussoir (40) comprend une plaque (45) montée sur un organe poussoir, ledit dispositif poussoir (40) est agencé en sorte de déplacer ladite plaque (45) en translation par rapport à la partie de toiture (30).

6. Dispositif (1) selon l'une ou l'autre des revendications 4 et 5, **caractérisé en ce que** le dispositif poussoir (40) est actionné par des moyens de commande permettant de pousser tout ou partie du volume de la matière organique (2) contenue dans la zone préliminaire de réception (35) ou contenue dans la zone préliminaire de chargement (37).

7. Dispositif (1) selon l'une quelconque des revendications 1 à 6, **caractérisé en ce que** la partie de toiture (30) de la zone préliminaire de réception (35) ou la partie de toiture (30) dans la zone préliminaire de chauffage (36) comprend une portion inclinée (48) vers la zone de collecte et d'alimentation (4).

8. Dispositif (1) selon la revendication 2, **caractérisé en ce que** les moyens de chauffage de la zone préliminaire de chauffage (36) comprennent des moyens d'alimentation en eau chaude, en particulier issue de la cogénération.

9. Procédé de méthanisation par voie sèche de matière organique (2), **caractérisé en ce qu'**il comprend les étapes suivantes :
a) une étape de réception de la matière organique (2) à digérer dans une zone préliminaire de réception;
b) une étape de transfert de la matière organique de la zone préliminaire de réception dans une zone de collecte et d'alimentation (4) ; et
c) une étape de transfert de la matière organique (2) de la zone de collecte et d'alimentation (4) vers un couloir de digestion (5) ayant au moins une ouverture d'admission (13) de la matière organique (2) et au moins une ouverture d'évacuation (14) de la matière organique (2,9), l'avancée de la matière organique (2) dans le couloir de digestion (5) entre les ouvertures d'admission (13) et d'évacuation (14) est obtenue par le déplacement gravitaire de la matière organique (2), le couloir de digestion (5) comprenant un dispositif de fermeture et d'ouverture (15) de ladite au moins une ouverture d'évacuation (14) ; et
d) une étape de collecte du biogaz produit ;
et **en ce que** le couloir de digestion comprend une partie de toiture agencée pour former le plancher de la zone préliminaire de réception de la matière organique, ladite zone de collecte et d'alimentation étant en contrebas de la partie de toiture en sorte que la matière organique puisse tomber, lors de son avancée dans la zone préliminaire de réception, de la zone préliminaire de réception dans la zone de collecte et d'alimentation.

10. Procédé selon la revendication 9, **caractérisé en ce que** la matière organique à digérer (2), en particulier dans le couloir de digestion, comprend au moins 30% de fumier en masse par rapport à sa masse totale, notamment au moins 50% de fumier en masse par rapport à sa masse totale, ledit fumier comprenant de la paille et/ou de la menue paille, éventuellement mélangée(s) avec du lisier et/ou des déjections animales.

11. Procédé selon l'une ou l'autre des revendications 9 et 10, **caractérisé en ce qu'**il comprend une étape d'arrosage de la matière organique disposée dans la zone préliminaire de réception à l'aide d'un fluide.

12. Procédé selon l'une quelconque des revendications 9 à 11, **caractérisé en ce qu'**il comprend une étape de chauffage préliminaire de la matière organique dans la zone préliminaire de réception, de préférence l'étape préliminaire de chauffage est effectuée après l'étape d'arrosage.

## Patentansprüche

1. Vorrichtung (1) zur Methanisierung von organischem Material (2) mittels eines Trockenverfahrens, umfassend wenigstens einen Fermenter (3), umfassend:
- mindestens eine Zone zum Sammeln und Zuführen (4) des zu fermentierenden organischen Materials (2),
- einen Fermentiergang (5), umfassend:
- eine Gaserzeugungszone (11), die dazu bestimmt ist, das organische Material (2) zu empfangen, das mit einem Fluid befeuchtet wurde, und
- eine Gassammelzone (12), die über der Gaserzeugungszone (11) in dem Fermentiergang (5) angeordnet ist, um das in der Gaserzeugungszone (11) produzierte Biogas zu sammeln,
**dadurch gekennzeichnet, dass** der Fermentiergang einen Dachteil (30) umfasst, der dazu eingerichtet ist, die Decke (32) einer Vorempfangszone (35) des organischen Materials zu bilden, wobei die Sammel- und Zuführzone (4) sich derart unterhalb des Dachteils (30) befindet, dass das organische Material bei seinem Vorrücken in die Vorempfangszone (35) von der Vorempfangszone (35) in die Sammel- und Zuführzone (4) fällt.

2. Vorrichtung (1) nach Anspruch 1, **dadurch gekennzeichnet, dass** die Vorempfangszone (35) eine Vorerwärmungszone (36) umfasst, die Erwärmungsmittel zur Erwärmung des organischen Materials (2) umfasst.

3. Vorrichtung (1) nach Anspruch 2, **dadurch gekennzeichnet, dass** die Vorempfangszone (35) eine Vorladezone (37) umfasst, und dadurch, dass die Vorerwärmungszone (36) mindestens eine Öffnung für den Einlass (38) des organischen Materials (2), das von der Vorladezone (37) stammt, und mindestens eine Öffnung für den Auslass (39) des organischen Materials (2) hin zu der Sammel- und Zuführzone (4) umfasst, wobei jede von der Einlass- (38) und der Auslassöffnung (39) Öffnungs- und Schließmittel umfasst.

4. Vorrichtung (1) nach einem der Ansprüche 1 bis 3, **dadurch gekennzeichnet, dass** die Vorempfangszone (35) eine Schiebevorrichtung umfasst, die eingerichtet ist, um das organische Material (2) in die Sammel- und Zuführzone (4) zu schieben (40) und um das organische Material (2), das in der Vorladezone (37) gelagert ist, in die Vorerwärmungszone (36) zu schieben, wobei das organische Material (2) entlang einer Verlagerungsrichtung A geschoben wird, die der Verlagerungsrichtung B des organischen Materials (2) in dem Fermentiergang entgegengesetzt ist.

5. Vorrichtung (1) nach Anspruch 4, **dadurch gekennzeichnet, dass** die Schiebevorrichtung (40) eine Platte (45) umfasst, die an einem Schiebeorgan montiert ist, wobei die Schiebevorrichtung (40) derart eingerichtet ist, dass sie die Platte (45) translatorisch in Bezug auf den Dachteil (30) verlagert.

6. Vorrichtung (1) nach einem der Ansprüche 4 und 5, **dadurch gekennzeichnet, dass** die Schiebevorrichtung (40) durch Steuermittel betätigt wird, die das Schieben des gesamten oder eines Teils des Volumens des organischen Materials (2), das in der Vorempfangszone (35) enthalten ist oder in der Vorladezone (37) enthalten ist, ermöglichen.

7. Vorrichtung (1) nach einem der Ansprüche 1 bis 6, **dadurch gekennzeichnet, dass** der Dachteil (30) der Vorempfangszone (35) oder der Dachteil (30) in der Vorerwärmungszone (36) einen hin zu der Sammel- und Zuführzone (4) geneigten Abschnitt (48) umfasst.

8. Vorrichtung (1) nach Anspruch 2, **dadurch gekennzeichnet, dass** die Erwärmungsmittel der Vorerwärmungszone (36) Mittel zur Zufuhr von Warmwasser umfassen, das insbesondere von der gleichzeitigen Erzeugung stammt.

9. Verfahren zur Methanisierung von organischem Material (2) mittels eines Trockenverfahrens, **dadurch gekennzeichnet, dass** es die folgenden Schritte umfasst:
a) einen Schritt des Empfangens des zu fermentierenden organischen Materials (2) in einer Vorempfangszone,
b) einen Schritt des Überführens des organischen Materials von der Vorempfangszone in eine Sammel- und Zuführzone (4) und
c) einen Schritt des Überführens des organischen Materials (2) von der Sammel- und Zuführzone (4) hin zu einem Fermentiergang (5), der mindestens eine Öffnung für den Einlass (13) des organischen Materials (2) und mindestens eine Öffnung für den Auslass (14) des organischen Materials (2, 9) aufweist, wobei das Vorrücken des organischen Materials (2) in dem Fermentiergang (5) zwischen der Einlass- (13) und der Auslassöffnung (14) durch die Verlagerung durch Schwerkraft des organischen Materials (2) erhalten wird, wobei der Fermentiergang (5) eine Vorrichtung zum Schließen und Öffnen (15) der mindestens einen Auslassöffnung (14) umfasst, und
d) einen Schritt des Sammelns des produzierten Biogases,
und dadurch, dass der Fermentiergang einen Dachteil umfasst, der eingerichtet ist, um die Decke der Vorempfangszone des organischen Materials zu bilden, wobei die Sammel- und Zuführzone sich derart unterhalb des Dachteils befindet, dass das organische Material bei seinem Vorrücken in die Vorempfangszone von der Vorempfangszone in die Sammel- und Zuführzone fallen kann.

10. Verfahren nach Anspruch 9, **dadurch gekennzeichnet, dass** das zu fermentierende organische Material (2) insbesondere in dem Fermentiergang mindestens 30 Masse-% Mist im Verhältnis zu seiner Gesamtmasse, insbesondere mindestens 50 Masse-% Mist im Verhältnis zu seiner Gesamtmasse, umfasst, wobei der Mist Stroh und/oder Spreu umfasst, das/die gegebenenfalls mit Gülle und/oder tierischen Exkrementen gemischt ist/sind.

11. Verfahren nach einem der Ansprüche 9 und 10, **dadurch gekennzeichnet, dass** es einen Schritt des Befeuchtens des in der Vorempfangszone angeordneten organischen Materials mittels eines Fluids umfasst.

12. Verfahren nach einem der Ansprüche 9 bis 11, **dadurch gekennzeichnet, dass** es einen Schritt des Vorerwärmens des organischen Materials in der Vorempfangszone umfasst, wobei der Schritt des Vorerwärmens vorzugsweise nach dem Schritt des Befeuchtens durchgeführt wird.

## Claims

1. A device (1) for the dry methanization of organic material (2), comprising at least one digester (3) comprising:
- at least one area for collecting and feeding (4) the organic matter (2) to be digested;
- a digestion corridor (5) comprising:
- a gas generating area (11) for receiving organic matter (2) that has been wetted with a fluid; and
- a gas collection area (12) disposed above the gas generation area (11) in said digestion corridor (5) for collecting the biogas produced in the gas generation area (11);
**characterized in that** the digestion corridor comprises a roof portion (30) arranged to form the floor (32) of a preliminary organic material receiving area (35), said collection and feeding area (4) being below the roof portion (30) so that organic material as it advances into said preliminary receiving area (35) falls from said preliminary receiving area (35) into the collection and feeding area (4).

2. The device (1) as claimed in claim 1, **characterized in that** the preliminary receiving area (35) comprises a preliminary heating area (36) comprising heating means for heating the organic material (2).

3. The device (1) as claimed in claim 2, **characterized in that** the preliminary receiving area (35) comprises a preliminary loading area (37), and **in that** the preliminary heating area (36) comprises at least one inlet opening (38) for organic material (2) from the preliminary loading area (37), and at least one outlet opening (39) for organic material (2) to the collection and feeding area (4), each of the inlet (38) and outlet (39) openings includes opening and closing means.

4. The device (1) as claimed in any one of claims 1 to 3, **characterized in that** the preliminary receiving area (35) comprises a pusher device arranged to push (40) the organic material (2) into the collection and feeding area (4) or to push the organic material (2) stored in the preliminary loading area (37) into the preliminary heating area (36), the organic material (2) being pushed in a direction of movement A opposite to the direction of movement B of the organic material (2) in the digestion corridor.

5. The device (1) as claimed in claim 4, **characterized in that** the pusher device (40) comprises a plate (45) mounted on a pusher member, said pusher device (40) is arranged to move said plate (45) in translation with respect to the roof portion (30).

6. The device (1) as claimed in either of claims 4 and 5, **characterized in that** the pusher device (40) is actuated by control means allowing to push all or part of the volume of organic material (2) contained in the preliminary receiving area (35) or contained in the preliminary loading area (37).

7. The device (1) as claimed in any one of claims 1 to 6, **characterized in that** the roof part (30) of the preliminary receiving area (35) or the roof part (30) in the preliminary heating area (36) comprises an inclined portion (48) towards the collection and feeding area (4).

8. The device (1) as claimed in claim 2, **characterized in that** the heating means of the preliminary heating area (36) comprise means for supplying hot water, in particular from cogeneration.

9. A process for the dry methanization of organic material (2), **characterized in that** it comprises the following steps:
a) a step of receiving the organic matter (2) to be digested in a preliminary receiving area;
b) a step of transferring organic matter from the preliminary receiving area to a collection and feeding area (4); and
c) a step of transferring organic matter (2) from the collection and feeding area (4) to a digestion corridor (5) having at least one organic matter (2) inlet opening (13) and at least one organic matter (2, 9) outlet opening (14), the advance of organic matter (2) into the digestion corridor (5) between the inlet opening (13) and outlet opening (14) is achieved by the gravitational displacement of the organic matter (2), the digestion corridor (5) comprising a device for closing and opening (15) said at least one outlet opening (14); and
d) a step of collecting the biogas produced;
and **in that** the digestion corridor includes a roof portion arranged to form the floor of the preliminary organic material receiving area, said collection and feeding area being below the roof portion so that organic material can fall, as it advances into the preliminary receiving area, from the preliminary receiving area into the collection and feeding area.

10. The process as claimed in claim 9, **characterized in that** the organic material to be digested (2), in particular in the digestion corridor, comprises at least 30% manure by mass based on its total mass, in particular at least 50% manure by mass based on its total mass, said manure comprising straw and/or small straw, optionally mixed with manure and/or animal manure.

11. The process as claimed in either of claims 9 and 10, **characterized in that** it comprises a step of spraying the organic material disposed in the preliminary receiving area with a fluid.

12. The process as claimed in any one of claims 9 to 11, **characterized in that** it comprises a step of preliminary heating of the organic material in the preliminary receiving area, preferably the preliminary heating step is carried out after the watering step.
